# EUROPEAN PATENT SPECIFICATION

(11) **EP 3 049 113 B1**
(45) Date of publication and mention of the grant of the patent: **27.12.2023**
(21) Application number: 14847993.4
(22) Date of filing: 26.09.2014
(51) Int. Cl.: A61K 39/395, A61K 39/40, A61K 39/00, A61K 39/38, A61K 39/02, A61K 39/108

(54) **BINDING MOIETIES FOR BIOFILM REMEDIATION**
BINDUNGSSTELLEN FÜR BIOFILMSANIERUNG
FRACTIONS DE LIAISON POUR DISSOLUTION DE BIOFILMS

(30) Priority: 13.01.2014 US 201461926828 P
(43) Date of publication of application: 03.08.2016
(60) Divisional application: 19219328.2
(73) Proprietor: Trellis Bioscience, LLC, Redwood City, CA 94063 (US)
(72) Inventor: KAUVAR, Lawrence M., San Francisco, CA 94109 (US); RYSER, Stefan, Menlo Park, CA 94025 (US); ESTELLES, Angeles, Belmont, CA 94002 (US); SIMON, Reyna J., Los Gatos, CA 95033 (US); BAKALETZ, Lauren Opremcak, Columbus, OH 43205 (US); GOODMAN, Steven David, Columbus, OH 43205 (US)
(74) Representative: V.O.
(86) International application number: PCT/US2014/057771
(87) International publication number: WO 2015/048484

(56) References cited:
- US-A1- 2005 049 402
- US-A1- 2011 236 306
- LAURA A. NOVOTNY ET AL: "Structural Stability of Burkholderia cenocepacia Biofilms Is Reliant on eDNA Structure and Presence of a Bacterial Nucleic Acid Binding Protein", PLOS ONE, vol. 8, no. 6, 1 January 2013 (2013-01-01) , page e67629, XP055147798, ISSN: 1932-6203, DOI: 10.1371/journal.pone.0067629
- GOODMAN S D ET AL: "Biofilms can be dispersed by focusing the immune system on a common family of bacterial nucleoid-associated proteins", MUCOSAL IMMUNOLOGY, NATURE PUBLISHING GROUP, US, vol. 4, no. 6, 1 November 2011 (2011-11-01), pages 625-637, XP009155507, ISSN: 1933-0219, DOI: 10.1038/MI.2011.27
- M. ELIZABETH BROCKSON ET AL: "Evaluation of the kinetics and mechanism of action of anti-integration host factor-mediated disruption of bacterial biofilms", MOLECULAR MICROBIOLOGY, 19 September 2014 (2014-09-19), pages n/a-n/a, XP055147799, ISSN: 0950-382X, DOI: 10.1111/mmi.12735
- LAURA A. NOVOTNY ET AL: "Monoclonal antibodies against DNA-binding tips of DNABII proteins disrupt biofilms in vitro and induce bacterial clearance in vivo", EBIOMEDICINE, vol. 10, 1 August 2016 (2016-08-01), pages 33-44, XP055352908, ISSN: 2352-3964, DOI: 10.1016/j.ebiom.2016.06.022
- C.J. ROCCO ET AL: "Natural antigenic differences in the functionally equivalent extracellular DNABII proteins of bacterial biofilms provide a means for targeted biofilm therapeutics", MOLECULAR ORAL MICROBIOLOGY, vol. 32, no. 2, 25 April 2016 (2016-04-25) , pages 118-130, XP055352918, ISSN: 2041-1006, DOI: 10.1111/omi.12157
- ANGELES ESTELLÉS ET AL: "A High-Affinity Native Human Antibody Disrupts Biofilm from Staphylococcus aureus Bacteria and Potentiates Antibiotic Efficacy in a Mouse Implant Infection Model", ANTIMICROBIAL AGENTS AND CHEMOTHERAPY, vol. 60, no. 4, 1 April 2016 (2016-04-01), pages 2292-2301, XP055318140, ISSN: 0066-4804, DOI: 10.1128/AAC.02588-15
- LAURA A. NOVOTNY ET AL: "Antibodies against the majority subunit of type IV pili disperse nontypeable H aemophilus influenzae biofilms in a LuxS-dependent manner and confer therapeutic resolution of experimental otitis media", MOLECULAR MICROBIOLOGY., vol. 96, no. 2, 15 February 2015 (2015-02-15), pages 276-292, XP055318148, GB ISSN: 0950-382X, DOI: 10.1111/mmi.12934
- SUN DAQIAN ET AL: "Inhibition of biofilm formation by monoclonal antibodies against Staphylococcus epidermidis RP62A accumulation-associated protein", CLINICAL AND VACCINE IMMUNOLOGY, AMERICAN SOCIETY FOR MICROBIOLOGY, US, vol. 12, no. 1, 1 January 2005 (2005-01-01), pages 93-100, ISSN: 1071-412X, DOI: 10.1128/CDLI.12.1.93-100.2005
- TABRIZI MOHAMMAD ET AL: "Biodistl'ibution Mechanisms of Therapeutic Monoclonal Antibodies in Health and Disease", THE AAPS JOUMAL, vol. 12, no. 1, 1 March 2009 (2009-03-01), pages 1-11, DOI: 10.1208/sl2248-()()9-9157-5
- XIONG YAN Q. ET AL: "A Human Biofilm-Disrupting Monoclonal Antibody Potentiates Antibiotic Efficacy in Rodent Models of both Staphylococcus aureus and Acinetobacter baumannii Infections", ANTIMICROBIAL AGENTS AND CHEMOTHERAPY, vol. 61, no. 10, 17 July 2017 (2017-07-17) , pages 1-10, US ISSN: 0066-4804, DOI: 10.1128/AAC.00904-17

## Description

### Technical Field

The invention relates to methods and compositions for dissolution of biofilms that inhibit immune responses and make bacteria resistant to antibiotics. More specifically, it concerns monoclonal antibodies that are derived from human cells or from transgenic animals expressing human antibody genes or that are humanized forms of antibodies native to other species wherein the affinity for the proteins that are responsible for the structural integrity of such biofilms exceeds the affinity of these proteins for biofilm components..

### Background Art

It is well understood in the art that bacterial infections may lead to formation of biofilms that protect the bacteria from the immune system and lead them to enter a quiescent, slow growth state that makes them resistant to most antibiotics (Donlan, R. M., et al., Clin Microbiol Rev (2002) 15:167-193). The result is persistent, recurrent infections that are very difficult to eliminate. These biofilms include as a major component branched DNA molecules that are held together by specific proteins generally designated DNABII proteins, with homologs found in most bacterial species (Goodman, S. D., et al., Mucosal Immunity (2011) 4:625-637). The substantial homology of these proteins facilitates the cooperative formation of biofilms, a feature that further renders the bacteria problematic from a treatment perspective. The present invention is based on the concept that supplying a binding moiety with sufficiently high affinity for this class of proteins will extract the proteins from the biofilm and thereby provide an effective method of destroying the biofilm by destroying the ability of the protein to bind and hold together the branched DNA. A supplied binding moiety against the DNABII protein may also destroy its ability to bind to other components present in the biofilm. Goodman et al. (supra) and Novotny et al. PLOS, Vol. 8, Nr 6, e67629, both disclose the treatment of biofilms by targeting DNAB with antiserum.

The binding moieties, of which monoclonal antibodies or fragments thereof are an important embodiment, can be supplied directly to biofilms or used to coat surfaces to provide an immuno-adsorbent for confining the DNABII protein(s). Applications include treatments of bacterial infections by systemic administration, subcutaneous, topical or inhaled administration, as well as reduction of biofouling that affects pipelines and other industrial equipment. Application to corresponding biofilm associated diseases of animals is also part of the present invention.

PCT publication WO2011/123396 provides an extensive discussion of such biofilms and suggests their removal by administering to a subject polypeptides that represent the DNABII protein itself, thus causing the organism to generate antibodies that can destroy the integrity of the biofilm. This document also suggests, in the alternative, supplying the antibodies themselves, either *ex vivo* to biofilms that exist outside an organism or to a subject to confer passive protection.

This PCT application describes the use of polyclonal antibodies generated against a particular DNABII protein (*E. coli* integration host factor (IHF)) to treat an animal model of the common ear infection (otitis media) and of an animal model for periodontal disease. It also describes generating active immunity by providing the protein, or peptides representing the protein to a subject. There is no disclosure of any monoclonal antibodies with the desired affinity that are directed to this protein. Nor is there any disclosure of binding moieties that show cross-species activity against homologs of the IHF protein. Achieving both properties represents a significant obstacle to discovery of an effective drug. The present invention overcomes these obstacles and provides improved agents for passive immunity.

### Disclosure of the Invention

The invention is defined in the claims. The invention provides homogeneous compositions of monoclonal antibodies comprising the variable region of the heavy chain of TRL1068 of SEQ ID NO:5 and further comprises the variable region of the light chain of TRL1068 of SEQ ID NO:6 or fragments thereof, that are particularly effective in binding the DNABII protein and thus effective in dissolving biofilms. Thus, the invention is directed to a monoclonal antibody (mAb) that has affinity for at least one DNABII protein that exceeds the affinity of branched DNA, a component of biofilms, for said protein. It is particularly preferred that any antibodies to be used systemically be compatible with mammalian subjects, especially human subjects or feline, canine, porcine, bovine, ovine, caprine or equine subjects when proposed for use in these subjects. Such native mAbs have lower risk of binding to other proteins in the body than mAbs from other sources and thus pose lower toxicity risk. Similarly, immunogenicity of mAbs native to a subject is expected to be lower than for other mAb sources thereby facilitating repeated administration. Specific binding moieties illustrated herein contain at least the CDR regions of the heavy chains, and optionally the light chains of the mAb's TRL295, TRL1012, TRL1068, TRL1070, TRL1087, TRL1215, TRL1216, TRL1218, TRL1230, TRL1232, TRL1242 and TRL1245. The present invention refers to the mAb TRL1068. However, other types of binding moieties, such as aptamers, modifications of antibodies such as camel type single-chain antibodies are also disclosed herein.

The invention is further directed to an *in vitro* method to treat a biofilm associated with an industrial process by using the binding moieties of the invention either to dissolve biofilms or prevent their formation. In this instance, the species origin of mAb's is not of concern. The binding moieties may also be applied topically on a subject to dissolve biofilms or to prevent their formation. The binding moieties may also be administered systematically for use in a treatment of biofilms.

In still other aspects, the invention is directed to recombinant materials and methods to prepare binding moieties of the invention that are proteins, and to improved recombinant methods to prepare DNABII proteins.

Further, novel expression systems for DNABII proteins to be used as immunogens are described and methods to use these DNABII proteins to identify an agent that reverses drug resistance in multiple species of bacteria. The latter methods comprise evaluating agents for binding activity to the DNABII proteins produced by multiple microbial species.

Moreover, specific isolated peptides are described that span predicted immunogenic epitope regions of the IHFα chain of the *E. coli* DNABII as well as methods for generating antibodies to IHF proteins by using these peptides as immunogens.

In still another aspect, the invention is directed to mAb of the present invention for use in a method to treat human or animal diseases for which biofilm causes drug resistance. Examples include: heart valve endocarditis (for which surgical valve replacement is required in the substantial fraction of cases that cannot be cured by high dose antibiotics due to the resistance associated with biofilm), chronic non-healing wounds (including venous ulcers and diabetic foot ulcers), ear and sinus infections, urinary tract infections, pulmonary infections (including subjects with cystic fibrosis or chronic obstructive pulmonary disease), catheter associated infections (including renal dialysis subjects), subjects with implanted prostheses (including hip and knee replacements), and periodontal disease. This mAb is effective in mammalian subjects in general, and thus is also u to household pets. For example, 85% of dogs over the age of 3 have periodontal disease which is difficult to treat due to biofilm (Kortegaard, H. E., et al., J. Small Anim. Pract. (2008) 49:610-616). Similarly, the invention has utility for treating farm animals. For example, 98% of cultures from dairy cattle with mastitis display bacterial infections associated with biofilm production: Poliana de Castro Melo, et al., Brazilian J. Microbiology (2013) 44:119-124).

### Brief Description of the Drawings

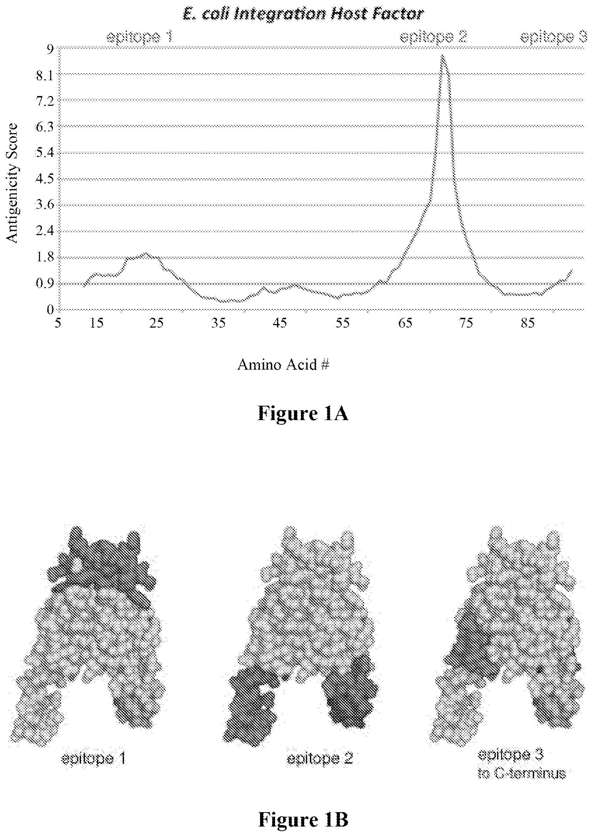

Figure 1A shows the result of a computational analysis of sites on IHF that are likely to be particularly susceptible to antibody attack (scores above 0.9). Residues 10-25, 56-78, and 86-96 of *Haemophilus influenzae* (Hi) IHF are thereby identified as promising targets. Figure 1B shows these likely antigenic sites mapped onto the crystal structure of the IHF protein (based on the Protein Data Bank (pdb) structure designated 1OWF).

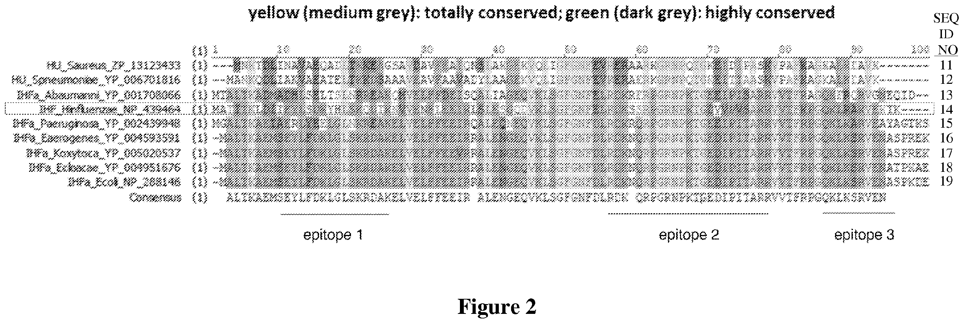

Figure 2 shows the location of the predicted epitopes of the invention in IHF proteins of various bacterial species.

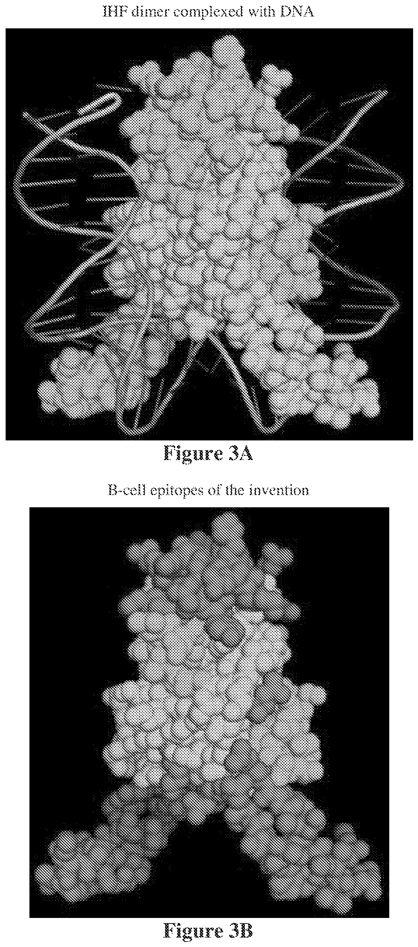

Figure 3A shows a three-dimensional model of IHF proteins in their native dimeric form as complexed with DNA. Figure 3B shows the predicted highly antigenic regions (the darkened regions shown (which are red in the color version). The epitopes 2 and 3 identified in Figure 1 are partially shielded from exposure to the immune system by DNA which is abundant in the biofilm.

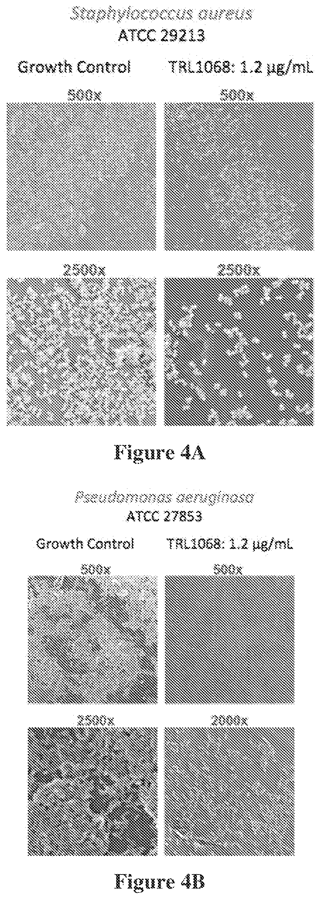

Figure 4A shows *Staphylococcus aureus* (Sa) biofilm treated for 12 hours with an isotype control mAb that does not bind the target protein (growth control) or with TRL1068 at 1.2 µg/mL (~10 nM), a native human mAb against a conserved epitope on DNABII proteins. TRL1068 caused dissolution of the biofilm, as evident at both low (500x) and high (2500x) magnification (scanning electron microscope images). Figure 4B shows the parallel experiment on *Pseudomonas aeruginosa* (Pa) biofilm.

### Modes of Carrying Out the Invention

The invention includes various binding moieties of a monoclonal or homogeneous nature comprising the variable region of the heavy chain of TRL1068 of SEQ ID NO:5 and further comprises the variable region of the light chain of TRL1068 of SEQ ID NO:6 that can dissolve biofilms. "Monoclonal" means that the binding moieties can form a homogeneous population analogous to the distinction between monoclonal and polyclonal antibodies. In one important embodiment, the exemplified binding moieties are mAbs or fragments thereof. In most embodiments, the binding moieties have affinity for at least one DNABII protein in the low nanomolar range - *i.e.,* the Kd is in the range of 10 nM - 100 nM including the intervening values, such as 25 nM or 50 nM, but may also be < 10 nM as a preferred embodiment.

As the illustrative antibodies disclosed herein in the examples below are derived from humans, the constant regions of these antibodies will be human which offers particular advantages for repeated use in humans. When the subject to be administered the mAb is non-human, it is advantageous for repeated use to administer native mAb's derived from that species. Alternatively, an equivalent of the human variable regions, optionally fused to an Fc region from the host species to be treated, may be used. This variable region may be, in some embodiments, an Fab portion or a single-chain antibody containing CDR regions from both the heavy and light chains. Bispecific forms of these variable regions equivalents can also be constructed, with numerous constructs described in the literature. Although the typical "mAb" will be a protein or polypeptide ("proteins," "polypeptide" and "peptide" are used interchangeably herein without regard to length) for use in subjects, the mAb's may also be supplied via delivery of nucleic acids that then generate the proteins *in situ.* In addition, nucleic acid molecules are described that mimic the binding characteristics of these polypeptides or proteins can be constructed - *i.e*., aptamers can be constructed to bind molecules that are identified as described below by their ability to mimic the binding moieties. Successful mimicry of these aptamers for the protein-based binding moieties can verified both biochemically and functionally to confirm that the affinity of the aptamer is sufficient for therapeutic efficacy.

With respect to protein-based monoclonal binding moieties, in addition to typical monoclonal antibodies or fragments thereof that are immunologically specific for the same antigen, various forms of other scaffolding, including single-chain antibody forms such as those derived from camel, llama or shark are described as well as antibody mimics based on other scaffolds such as fibronectin, lipocalin, lens crystallin, tetranectin, ankyrin, Protein A (Ig binding domain), or the like. Short structured peptides are described which provide sufficient affinity and specificity, e.g. peptides based on inherently stable structures such as conotoxins or avian pancreatic peptides, or peptidomimetics that achieve stable structures by crosslinking and/or use of non-natural amino acids: Josephson K., et al., JAm Chem Soc (2005) 127(33):11727-11725). In general, "monoclonal antibody (mAb)" includes all of the foregoing.

As used herein, the term "antibody" includes immunoreactive fragments of traditional antibodies even if, on occasion, "fragments" are mentioned redundantly. The antibodies, thus, include Fab, F(ab')₂, Fᵥ fragments, single-chain antibodies which contain a substantially only variable regions, bispecific antibodies and their various fragmented forms that still retain immunospecificity and proteins in general that mimic the activity of "natural" antibodies by comprising amino acid sequences or modified amino acid sequences (*i.e*., pseudopeptides) that approximate the activity of variable regions of more traditional naturally occurring antibodies.

For the variable regions of mAb's, as is well known, the critical amino acid sequences are the CDR sequences arranged on a framework which framework can vary without necessarily affecting specificity or decreasing affinity to an unacceptable level. Definition of these CDR regions is accomplished by art-known methods. Specifically, the most commonly used method for identifying the relevant CDR regions is that of Kabat as disclosed in Wu, T. T., et al., J. Exp. Med. (1970) 132:211-250 and in the book Kabat, E. A., et al. (1983) Sequence of Proteins of Immunological Interest, Bethesda National Institute of Health, 323 pages. Another similar and commonly employed method is that of Chothia, published in Chothia, C., et al., J. Mol. Biol. (1987) 196:901-917 and in Chothia, C., et al., Nature (1989) 342:877-883. An additional modification has been suggested by Abhinandan, K. R., et al., Mol. Immunol. (2008) 45:3832-3839. The present invention includes the CDR regions as defined by any of these systems or other recognized systems known in the art.

The specificities of the binding of the mAb's of the invention are defined, as noted, by the CDR regions mostly those of the heavy chain, but complemented by those of the light chain as well (the light chains being somewhat interchangeable). Therefore, the mAb's of the invention contain the three CDR regions of a heavy chain and the three CDR's of a light chain that matches it. Because binding affinity is also determined by the manner in which the CDR's are arranged on a framework, the mAb's of the invention contain complete variable regions of the heavy chain containing the three relevant CDR's as well as, the complete light chain variable region comprising the three CDR's associated with the light chain complementing the heavy chain in question. This is true with respect to the mAb's that are immunospecific for a single epitope as well as for bispecific antibodies or binding moieties that are able to bind two separate epitopes, for example, divergent DNABII proteins from two bacterial species.

The mAb's of the invention may be produced recombinantly using known techniques. Thus, with regard to the novel antibodies described herein, the invention also relates to nucleic acid molecules comprising nucleotide sequence encoding them, as well as vectors or expression systems that comprise these nucleotide sequences, cells containing expression systems or vectors for expression of these nucleotide sequences and methods to produce the binding moieties by culturing these cells and recovering the binding moieties produced. Any type of cell typically used in recombinant methods can be employed including prokaryotes, yeast, mammalian cells, insect cells and plant cells. Also included are human cells (*e.g.,* muscle cells or lymphocytes) transformed with a recombinant molecule that encodes the novel antibodies.

Bispecific binding moieties may be formed by covalently linking two different binding moieties with different specificities. For example, the CDR regions of the heavy and light chain derived from one monospecific mAb may be coupled through any suitable linking means to peptides comprising the CDR regions of the heavy chain sequence and optionally light chain of a second mAb. If the linkage is through an amino acid sequence, the bispecific binding moieties can be produced recombinantly and the nucleic acid encoding the entire bispecific entity expressed recombinantly. As was the case for the binding moieties with a single specificity, the invention also includes the possibility of binding moieties that bind to one or both of the same epitopes as the bispecific antibody or binding entity/binding moiety that actually contains the CDR regions.

The invention includes bispecific constructs which comprise the complete heavy and light chain sequences or the complete heavy chain sequence and at least the CDR's of the light chains or the CDR's of the heavy chains and the complete sequence of the light chains.

The invention is also directed to nucleic acids encoding the bispecific moieties and to recombinant methods for their production.

Multiple technologies now exist for making a single antibody-like molecule that incorporates antigen specificity domains from two separate antibodies (bi-specific antibody). Thus, a single antibody with very broad strain reactivity can be constructed using the Fab domains of individual antibodies with broad reactivity to Group 1 and Group 2 respectively. Suitable technologies have been described by Macrogenics (Rockville, MD), Micromet (Bethesda, MD) and Merrimac (Cambridge, MA). (See, *e.g.,* Orcutt, K. D., Ackerman, M. E., Cieslewicz, M., Quiroz, E., Slusarczyk, A. L., Frangioni, J. V., Wittrup, K. D., "A Modular IgG-scFv Bispecific Antibody Topology," Protein Eng Des Sel. (2010) 23:221-228; Fitzgerald, J., Lugovskoy, A., "Rational Engineering of Antibody Therapeutics Targeting Multiple Oncogene Pathways," MAbs. (2011) 1:3(3); Baeuerle, P. A., Reinhardt, C., "Bispecific T-cell Engaging Antibodies for Cancer Therapy," Cancer Res. (2009) 69:4941-4944.)

A method is disclosed for identifying suitable immunogens for use to generate antibodies by assessing the binding of the binding moieties of the invention, such as mAb's described above, to a candidate peptide or other molecule. This is an effective method, not only to identify suitable immunogens, but also to identify compounds that can be used as a basis for designing aptamers that mimic binding moieties. The method is grounded in the fact that if a vaccine immunogen cannot bind to an optimally effective mAb, it is unlikely to be able to induce such antibodies. Conversely, an immunogen that is a faithful inverse of the optimal mAb provides a useful template for constructing a mimic of the optimal mAb. In its simplest form, this method employs a binding moiety such as one of the mAb's of the invention as an assay component and tests the ability of the binding moiety to bind to a candidate immunogen in a library of said candidates. Successful binders can be used to generate additional antibodies for treating biofilms by administering them to vertebrate subjects such as mice, rabbits, rats, other mammals or avian subj ects.

In addition, the ability of the binding moieties of the invention to overcome drug resistance in a variety of bacteria can be assessed by testing the binding moieties of the invention against a panel or library of DNABII proteins from a multiplicity of microbial species. Binding moieties that are able to bind effectively a multiplicity of such proteins are thus identified as suitable not only for dissolving biofilms in general, but also as effective against a variety of microbial strains. It is also useful to identify binding moieties that have utility in acidic environments wherein the affinity of a candidate binding moiety for a DNABII protein over a range of pH conditions is tested and moieties with a low nanomolar affinity at pH 4.5 are identified as having utility in acidic environments.

The binding moieties of the invention are also verified to have an affinity with respect to at least one DNABII protein greater than the affinity of a biofilm component for the DNABII protein which comprises comparing the affinity of the binding moiety for the DNABII protein *versus* the affinity of a component of the biofilm, typically branched DNA, for the DNABII protein. This can be done in a competitive assay, or the affinities can be determined independently.

The DNABII proteins used in these assays may be prepared in mammalian cells at relatively high yield.

All of the assays above involve assessing binding of two perspective binding partners in a variety of formats.

A multitude of assay types are available for assessing successful binding of two prospective binding partners. For example, one of the binding partners can be bound to a solid support and the other labeled with a radioactive substance, fluorescent substance or a colorimetric substance and the binding of the label to the solid support is tested after removing unbound label. The assay can, of course, work either way with the binding moiety attached to the solid support and a candidate immunogen or DNABII protein labeled or *vice versa* where the candidate is bound to solid support and the binding moiety is labeled. Alternatively, a complex could be detected by chromatographic means based on molecular weight such as SDS-page. The detectable label in the context of the binding assay can be added at any point. Thus, if, for example, the mAb or other binding moiety is attached to a solid support the candidate immunogen can be added and tested for binding by supplying a labeled component that is specific for the candidate immunogen. Hundreds of assay formats for detecting binding are known in the art, including, in the case where both components are proteins, the yeast two-hybrid assay.

In addition to this straightforward application of the utility of the binding moieties of the invention, the identification of a suitable powerful immunogen can be determined in a more sophisticated series of experiments wherein a panel of mAbs against the DNABII protein is obtained and ranked in order by efficacy. A full suite of antibodies or other binding moieties can be prepared against all possible epitopes by assessing whether additional binding moieties compete for binding with the previous panel of members. The epitopes for representative binding mAbs for each member of the complete suite can be accomplished by binding to a peptide array representing the possible overlapping epitopes of the immunogen or by X-ray crystallography, NMR or cryoelectron microscopy. An optimal vaccine antigen would retain the spatial and chemical properties of the optimal epitope defined as that recognized by the most efficacious mAbs as compared to less efficacious mAbs but does not necessarily need to be a linear peptide. It may contain non-natural amino acids or other crosslinking motifs.

Moreover, screening can include peptides selected based on their likelihood of being recognized by antibodies and based on their conservation across bacterial species. As described in Example 3 below, for IHF these two criteria have converged on a single peptide - residues 56-78 of *H. influenzae* and corresponding positions in other analogs.

Thus, even beyond the specific mAb's set forth herein, optimal immunogens can be obtained, which not only are useful in active vaccines, but also as targets for selecting aptamers. Specifically, in addition to positions 56-78 of *H. influenzae,* the peptides at positions 10-25 and 86-96 of *H. influenzae* are identified.

A method is described to prepare higher yields of the bacterial/microbial DNABII proteins which are typically somewhat toxic to bacteria. The standard method for preparation of these proteins is described by Nash, H. A., et al., J. Bacteriol (1987) 169:4124-4127 who showed that the IHF of *E. coli* could be effectively prepared if both chains of said protein (IHF alpha and IHF beta) are produced in the same transformant. Applicants have found that they are able to obtain higher yields, as much as 5-10 mg/l of IHF, by producing both chains transiently in HEK293 cells. The expression of bacterial proteins that are toxic at high levels in bacteria is conveniently achieved in mammalian cells especially for those without glycosylation sites that would result in modification of the proteins when thus expressed. Purification of the resulting protein can be achieved using fast protein liquid chromatography (FPLC).

### Applications

The binding moieties of the invention including antibodies are useful in therapy and prophylaxis for any subject that is susceptible to infection that results in a biofilm. Thus, various mammals, such as bovine, ovine and other mammalian subjects including horses and household pets and humans will benefit from the prophylactic and therapeutic use of these mAb's.

The binding moieties of the invention may be administered in a variety of ways. Liposomal compositions are particularly useful, as are compositions that comprise micelles or other nanoparticles of various types. Further, the binding agent may be conjugated to any of the solid supports known in the literature, such as PEG, agarose or a dextran, to function as an immuno-sorbent for extracting IHF from a biofilm. Alternatively, the peptide-based mAb's may be administered as the encoding nucleic acids either as naked RNA or DNA or as vector or as expression constructs. The vectors may be non-replicating viral vectors such as adenovirus vectors (AAV) or the nucleic acid sequence may be administered as mRNA packaged in a liposome or other lipid particle. Use of nucleic acids as drugs as opposed to their protein counterparts is helpful in controlling production costs.

These are administered in a variety of protocols, including intravenous, subcutaneous, intramuscular, topical (particularly for chronic non-healing wounds and periodontal disease), inhaled and oral or by suppository. Similar routes of administration can be used with regard to the binding moieties themselves.

The peptides that represent the epitopes of the IHF proteins as described herein are useful as active components of vaccines to stimulate immunogenic responses which will generate antibodies *in situ* for disruption of biofilms. The types of administration of these immunogens or peptidyl mimetics that are similarly effective are similar to those for the administration of binding moieties, including various types of antibodies, etc. The peptidomimetics may themselves be in the form of aptamers or alternative structures that mimic the immunogenic peptides described herein. For those immunogens, however, that are proteins or peptides, the administration may be in the form of encoding nucleic acids in such form as will produce these proteins *in situ.* The formulation, routes of administration, and dosages are determined conventionally by the skilled artisan.

The types of conditions for which the administration either of the vaccine type for active generation of antibodies for biofilm control or for passive treatment by administering the antibodies *per se,* include any condition that is characterized by or associated with the formation of biofilms. These conditions include: heart valve endocarditis, both native and implanted (for which a substantial fraction of cases cannot be cured by high dose antibiotics due to the resistance associated with biofilm), chronic non-healing wounds (including venous ulcers and diabetic foot ulcers), ear and sinus infections, urinary tract infections, pulmonary infections (including subjects with cystic fibrosis or chronic obstructive pulmonary disease), catheter associated infections (including renal dialysis subjects), subjects with implanted prostheses (including hip and knee replacements), and periodontal disease.

As noted above, the binding moieties of the invention are not limited in their utility to therapeutic (or diagnostic) uses, but can be employed in any context where a biofilm is a problem, such as pipelines or other industrial settings. The mode of application of these binding moieties to the biofilms in these situations, again, is conventional.

The following examples are offered to illustrate but not to limit the invention which is directed to mAb TRL1068.

### Example 1

### Preparation of Antibodies

Human peripheral antibody producing memory B cells were obtained from recovered sepsis patients or from anonymized blood bank donors, under informed consent. The cells were subjected to the CellSpot^{™} assay to determine their ability to bind the DNABII protein derived from influenza virus. The CellSpot^{™} assay is described in U.S. patents 7,413,868 and 7,939,344. After isolating the B cells from whole blood, they were stimulated with cytokines and mitogens to initiate a brief period of proliferation and antibody secretion (lasting ~10 days) and plated for subjection to the assays; the encoding nucleic acids were extracted and used to produce the antibodies recombinantly.

Antibodies selected based on binding to at least one of the DNABII proteins or fragments thereof were characterized: TRL295, TRL1012, TRL1068, TRL1070, TRL1087, TRL1215, TRL1216, TRL1218, TRL1230, TRL1232, TRL1242 and TRL1245. Affinity was measured using the FortéBio^{™} Octet^{™} biosensor to measure on and off rates (whose ratio yields the Kd). This result establishes the feasibility of a focused screen to isolate high affinity, crossstrain binding antibodies.
TRL295 heavy chain variable region has the amino acid sequence: 
QVQLVESGGGLVQPGGSLRLSCAASGFPFSSYAMSWVRQAPGKGLEWVSAISGNGADSY
YADSVKGRFTTSRDKSKNTVYLQMNRLRAEDTAVYYCAKDMRRYHYDSSGLHFWGQG
TLVTVSS (SEQ ID NO:1);
TRL295 light chain variable region has the amino acid sequence: 
DIELTQAPSVSVYPGQTARITCSGDALPKQYAYWYQQKPGQAPVVVIYKDSERPSGISERF
SGSSSGTTVTLTISGVQAGDEADYYCQSVDTSVSYYVVFGGGTKLTVL (SEQ ID NO:2);
TRL1012 heavy chain variable region has the amino acid sequence: 
QVQLVESGGGLVQPGGSLRLSCAASGFPFSSYAMSWVRQAPGKGLEWVSAISGNGADSY
YADSVKGRFTTSRDKSKNTVYLQMNRLRAEDTAVYYCAKDMRRYHYDSSGLHFWGQG
TLVTVSS (SEQ ID NO:3);
TRL1012 light chain variable region has the amino acid sequence: 
DIMLTQPPSVSAAPGQKVTISCSGSSSNIGTNYVSWFQQVPGTAPKFLIYDNYKRPSETPDR
FSGSKSGTSATLDITGLQTGDEANYYCATWDSSLSAWVFGGGTKVTVL (SEQ ID NO:4);
TRL1068 heavy chain variable region has the amino acid sequence: 
QVQLVESGPGLVKPSETLSLTCRVSGDSNRPSYWSWIRQAPGKAMEWIGYVYDSGVTIYN
PSLKGRVTISLDTSKTRFSLKLTSVIAADTAVYYCARERFDRTSYKSWWGQGTQVTVSS
(SEQ ID NO:5);
TRL1068 light chain variable region has the amino acid sequence: 
DIVLTQAPGTLSLSPGDRATLSCRASQRLGGTSLAWYQHRSGQAPRLLILYGTSNRATDTPD
RFSGSGSGTDFVLTISSLEPEDFAVYYCQQYGSPPYTFGQGTTLDIK (SEQ ID NO:6);
TRL1070 heavy chain variable region has the amino acid sequence: 
QVQLVQSGGTLVQPGGSLRLSCAASGFTFSYYSMSWVRQAPGKGLEWVANIKHDGTERN
YVDSVKGRFTISRDNSEKSLYLQMNSLRAEDTAVYYCAKYYYGAGTNYPLKYWGQGTR
VTVSS (SEQ ID NO:7);
TRL1070 light chain kappa variable region has the amino acid sequence: 
DILMTQSPSSLSASVGDRVTITCRASQGIRNDLGWYQQKPGKAPKLLIYAASSLQSGVPSRF
SGSGSGTDFTLTISSLQPEDFATYYCLQDYNYPLTFGGGTKVEIKR (SEQ ID NO:8);
TRL1087 heavy chain variable region has the amino acid sequence: 
QVQLLESGPGLVRPSDTLSLTCTFSADLSTNAYWTWIRQPPGKGLEWIGYMSHSGGRDYN
PSFNRRVTISVDTSKNQVFLRLTSVTSADTAVYFCVREVGSYYDYWGQGILVTVSS
(SEQ ID NO:9);
TRL1087 light chain kappa variable region has the amino acid sequence: 
DIEMTQSPSSLSASVGDRITITCRASQGISTWLAWYQQKPGKAPKSLIFSTSSLHSGVPSKFS
GSGSGTDFTLTITNLQPEDFATYYCQQKWETPYSFGQGTKLDMIR (SEQ ID NO:10);
TRL1215 heavy chain variable region has the amino acid sequence: 
QVQLVESGTEVKNPGASVKVSCTASGYKFDEYGVSWVRQSPGQGLEWMGWISVYNGKT
NYSQNFQGRLTLTTETSTDTAYMELTSLRPDDTAVYYCATDKNWFDPWGPGTLVTVSS
(SEQ ID NO:11);
TRL1215 light chain lambda variable region has the amino acid sequence: 
DIVMTQSPSASGSPGQSITISCTGTNTDYNYVSWYQHHPGKAPKVIIYDVKKRPSGVPSRFS
GSRSGNTATLTVSGLQTEDEADYYCVSYADNNHYVFGSGTKVTVL (SEQ ID NO:12);
TRL1216 heavy chain variable region has the amino acid sequence: 
QVQLVESGGGVVQPGGSLRVSCAASAFSFRDYGIHWVRQAPGKGLQWVAVISHDGGKKF
YADSVRGRFTISRDNSENTLYLQMNSLRSDDTAVYYCARLVASCSGSTCTTQPAAFDIWG
PGTLVTVSS (SEQ ID NO:13);
TRL1216 light chain lambda variable region has the amino acid sequence: 
DIMLTQPPSVSVSPGQTARITCSGDALPKKYTYWYQQKSGQAPVLLIYEDRKRPSEIPERFS
AFTSWTTATLTITGAQVRDEADYYCYSTDISGDIGVFGGGTKLTVL (SEQ ID NO:14);
TRL1218 heavy chain variable region has the amino acid sequence: 
QVQLLESGADMVQPGRSLRLSCAASGFNFRTYAMHWVRQAPGKGLEWVAVMSHDGYT
KYYSDSVRGQFTISRDNSKNTLYLQMNNLRPDDTAIYYCARGLTGLSVGFDYWGQGTLV
TVSS (SEQ ID NO:15);
TRL1218 light chain lambda variable region has the amino acid sequence: 
DIVLTQSASVSGSPGQSITISCTGTSSDVGGYNYVSWYQQHPGKAPKLMIYDVTTRPSGVS
DRFSGSKSGNTASLTISGLQAEDEADYYCSSYSSGSTPALFGGGTQLTVL (SEQ ID NO:16);
TRL1230 heavy chain variable region has the amino acid sequence: 
QVQLVQSGGGLVKPGGSLRLSCGASGFNLSSYSMNWVRQAPGKGLEWVSSISSRSSYIYY
ADSVQGRFTISRDNAKNSLYLQMNSLRAEDTAIYYCARVSPSTYYYYGMDVWGQGTTVT
VSS (SEQ ID NO:17);
TRL1230 light chain lambda variable region has the amino acid sequence: 
DIVLTQPSSVSVSPGQTARITCSGDELPKQYAYWYQQKPGQAPVLVIYKDNERPSGIPERFS
GSSSGTTVTLTISGVQAEDEADYYCQSADSSGTYVVFGGGTKLTVL (SEQ ID NO:18);
TRL1232 heavy chain variable region has the amino acid sequence: 
QVQLVESGAEVKKPGALVKVSCKASGYTFSGYYMHWVRQAPGQGLEWMGWINPKSGG
TKYAQKFQGRVTMTRDTSISTAYMELSRLRSDDTAVYFCARGGPSNLERFLERLQPRYSY
DDKYAMDVWGQGTTVTVSS (SEQ ID NO:19);
TRL1232 light chain kappa variable region has the amino acid sequence: 
DIVMTQSPGTLSLSPGARATLSCRASQSVSSIYLAWYQQKPGQAPRLLIFGASSRATGIPDR
FSGSGSGTDFTLTISRLEPEDFAVYYCQQYGSSPYTFGQGTKLEIKR (SEQ ID NO:20);
TRL1242 heavy chain variable region has the amino acid sequence: 
QVQLVQSGTEVKKPGESLKISCEGSRYNFARYWIGWVRQMPGKGLDWMGIIYPGDSDTR
YSPSFQGQVSISADKSISTAYLQWNSLKASDTAMYYCARLGSELGVVSDYYFDSWGQGTL
VTVSS (SEQ ID NO:21);
TRL1242 light chain kappa variable region has the amino acid sequence: 
DIVLTQSPDSLAVSLGERATINCKSSQSVLDRSNNKNCVAWYQQKPGQPPKLLIYRAATRE
SGVPDRFSGSGSGTDFSLTISSLQAEDVAVYFCQQYYSIPNTFGQGTKLEIKR
(SEQ ID NO:22); and
TRL1245 heavy chain variable region has the amino acid sequence: 
QVQLVESGGGLVKAGGSLRLSCVASGFTFSDYYMSWIRQAPGKGLEWISFISSSGDTIFYA
DSVKGRFTVSRDSAKNSLYLQMNSLKVEDTAVYYCARKGVSDEELLRFWGQGTLVTVSS
(SEQ ID NO:23);
TRL1245 light chain variable region has the amino acid sequence:

DIVLTQDPSVSVSPGQTARITCSGDALPKKYAYWYQQKSGQAPVLVIYEDTKRPSGIPERF
SGSSSGTVATLTISGAQVEDEADYYCYSTDSSGNQRVFGGGTKLTVL (SEQ ID NO:24).

The encoding nucleotide sequences for the variable regions of TRL295 are:

Heavy Chain: caggtgcagctggtgcagtctgggggaggcttggtacagcctggggggtccctgagactctcctgtgcagcct
ctggatttaccttcagtgattatagtatgaactgggtccgccaggctccagggaagggactggaatggctttcatacattagtcacactattactac
catatactacgccgactctgtgaagggccgattcaccatctccagagacaatgccgacagctcactgtatctccaaatgaacagcctgggaga

cgaggacacggctgtgtattactgtgcgagagctccattagtaaactgtagtactagtggctgccagtccggaagctggttcgacacctggggc
cagggaaccctggtcaccgtctcctca (SEQ ID NO:25);

Light Chain: gatatcgagctgactcaggcaccctcggtgtcagtgtatccaggacagacggccaggatcacctgctctggaga
tgcactgccaaagcaatatgcttattggtaccagcagaagccaggccaggcccctgtggtggtgatatataaagacagtgagaggccctcagg
gatctctgagcgattctctggctccagctcggggacaacagtcacgttgaccatcagtggagtccaggcaggggacgaggctgactattattgt
caatcagttgacaccagtgtttcttattatgtggtcttcggcggagggaccaagttgaccgtccta (SEQ ID NO:26).

The encoding nucleotide sequences for the variable regions of TRL1012 are:

Heavy Chain: caggtgcagctggtggagtccgggggaggcttggtacagcctggggggtccctgagactttcctgtgccgcc
tctggattccccttcagtagttatgccatgagttgggtccgtcaggctccagggaaggggctggagtgggtctcagccatcagtggcaacggc
gctgactcatattacgcagactccgtgaagggccgcttcaccacttccagagacaagtccaagaatacagtttatttgcaaatgaacagactcag
ggccgaggacacggccgtatattactgtgcgaaagatatgcgacggtatcattatgacagtagtggtctgcacttctggggccagggaaccct
ggtcaccgtctcctca (SEQ ID NO:27);

Light Chain: gatatcatgctgactcagcccccctcagtgtctgcggcccccggacagaaggtcaccatctcctgctctggaagc
agctccaacattgggacgaattatgtgtcctggttccagcaggtcccaggaacagcccccaaattcctcatttatgacaattataaacgaccctca
gaaactcctgaccgattctctggctccaagtctggcacgtcggccaccctggacatcaccggactccagactggggacgaggccaattattac
tgcgcaacatgggacagtagcctgagtgcttgggtgttcggcggagggaccaaggtgaccgtcctg (SEQ ID NO:28).

The encoding nucleotide sequences for the variable regions of TRL1068 are:

Heavy Chain: caggtgcagctggtggagtccggcccaggactggtgaagccttcggagaccctgtccctcacctgcagggtc
tctggtgactccaatcggccttcctactggagctggatcaggcaggccccagggaaggcaatggagtggataggttatgtctatgacagtggg
gtcaccatctacaatccctccctcaagggtcgagtcacaatatcactagacacgtcgaagacgcggttctccctgaaactgacctctgtgatcgc
tgcggacacggccgtatattattgtgcgcgagaacgttttgatcggacatcgtataagagttggtggggccagggaacgcaggtcaccgtctcc
tca (SEQ ID NO:29);

Light Chain: gatatcgtgctgactcaggccccaggcactctgtctttgtctccaggggacagagccaccctctcctgtagggcc
agtcagcgtcttggcggcacgtccttagcctggtaccagcacagatctggccaggctcccaggctcatcctctacggaacttcaaacagggcc
actgacacccctgacaggtttagtggcagtgggtctgggacagacttcgttctcaccatcagttccctggagcctgaagattttgcagtgtattact
gtcagcaatatggcagcccaccgtacacttttggccaggggaccactctggacatcaaa (SEQ ID NO:30).

The encoding nucleotide sequences for the variable regions of TRL1070 are:

Heavy Chain: caggtgcagctggtgcagtctgggggaaccttggtccagccgggggggtccctgagactctcctgtgcagcc
tctggattcacctttagttactactcgatgagctgggtccgccaggctccagggaaggggctggagtgggtggccaacataaagcacgatgga
actgagagaaattatgtggactctgtgaagggccgattcaccatctccagagacaacagcgagaagtctctttacctgcaaatgaacagcctga
gagccgaggacacggctgtgtattactgtgcgaagtattattatggtgccgggactaattatccccttaagtactggggccagggaacccgggt
caccgtctcctca (SEQ ID NO:31);

Light Chain: gatatcctgatgacccagtctccatcctccctgtctgcatctgtaggagacagagtcaccatcacttgccgggcaa
gtcagggcattagaaatgatttaggctggtatcagcagaaaccagggaaagcccctaagctcctgatctatgctgcatccagtttacaaagtgg
ggtcccatcaaggttcagcggcagtggatctggcacagatttcactctcaccatcagcagcctgcagcctgaagattttgcaacttattactgtct
acaagattacaattacccgctcactttcggcggagggaccaaggtggagatcaaacga (SEQ ID NO:32).

The encoding nucleotide sequences for the variable regions of TRL1087 are:

Heavy Chain: caggtgcagctgctcgagtcaggcccaggcctggttaggccctcggacaccctgtccctcacctgcactttttc
cgctgacctcagcaccaacgcctattggacctggatccggcagcccccaggaaagggactggagtggattggctatatgtctcatagtgggg
gaagggattacaatccctccttcaaccggcgagtcaccatttcagtggacacgtcgaagaaccaggttttcttgaggctgacgtcagtgacctct
gcggacacggccgtctatttctgtgtgagagaagtcggcagttactacgactactggggccagggaatcctggtcaccgtctcctca
(SEQ ID NO:33);

Light Chain: gatatcgagatgacccagtctccatcctctttgtctgcatctgtcggagacagaatcaccatcacttgtcgggcga
gtcagggtattagcacctggttagcctggtatcagcagaaaccggggaaagcccctaagtccctgatcttttctacgtccagcctgcatagtggg
gtcccctcaaagttcagcggcagtgggtctgggacagacttcactctcaccatcaccaacctgcagcctgaagattttgcaacttattactgcca
acagaaatgggagaccccttatagttttggccaggggaccaagctggacatgatacga (SEQ ID NO:34).

The encoding nucleotide sequences for the variable regions of TRL1215 are:

Heavy Chain: caggtgcagctggtggagtctggaactgaggtgaagaaccctggagcctcagtgaaggtctcctgcacggcc
tctggttacaaatttgacgaatatggtgtcagttgggtgcgacagtcccctggacaaggacttgagtggatgggatggatcagtgtttataatggc
aagacaaactatagccagaactttcagggcagactcaccctgaccacagagacatccaccgacacagcctacatggagcttacgagcctcag
acctgacgacacggccgtctattactgtgcgacagacaaaaactggttcgacccctggggcccgggaaccctggtcaccgtctcctca
(SEQ ID NO:35);

Light Chain: gatatcgtgatgacccagtctccctccgcgtccgggtctcctggacagtcaatcaccatctcctgcactggaacca
acactgattataattatgtttcctggtaccagcaccaccccggcaaagcccccaaagtcattatttatgacgtcaaaaagcggccctcgggggtc
cctagtcgcttctctggctccaggtctggcaacacggccaccctgaccgtctctgggctccagactgaggatgaggctgattattattgtgtctca
tatgcagacaacaatcattatgtcttcggaagtgggaccaaggtcaccgtcctg (SEQ ID NO:36).

The encoding nucleotide sequences for the variable regions of TRL1216 are:

Heavy Chain: caggtgcagctggtggagtccgggggaggcgtggtccagcctggagggtccctgagagtctcctgtgcagc
ctctgcgttcagtttcagggattatggcatacactgggtccgccaggctccaggcaaggggctgcaatgggtggcggttatttcacatgatgga
ggtaagaaattctatgcagactccgtgaggggccgattcaccatctccagagacaattccgagaacacactgtatctccaaatgaacagcctga
gatctgacgacacggctgtctattactgtgcgaggctcgttgccagttgcagtggttccacctgcacaacgcaacctgctgcctttgacatttggg
gcccagggacattggtcaccgtctcttca (SEQ ID NO:37);

Light Chain: gatatcatgctgactcagccgccctcggtgtcagtgtccccaggacaaacggccaggatcacctgctctggaga
tgcattgccaaaaaaatatacttattggtatcagcagaagtcaggccaggcccctgttctgctcatctatgaggacaggaaacgaccctccgag

atccctgagagattctctgccttcacctcatggacgacggccaccttgactatcactggggcccaggtgagagatgaagctgactactactgtta
ttcaacagacatcagtggtgatataggagtgttcggcggagggaccaagctgaccgtccta (SEQ ID NO:38).

The encoding nucleotide sequences for the variable regions of TRL1218 are:

Heavy Chain: gatatcgtgctgactcagtcggcctccgtgtctgggtctcctggacagtcgatcaccatctcctgcactggaacc
agcagtgacgttggtggatataactatgtctcctggtaccaacaacacccaggcaaagcccccaaactcatgatttatgatgtcactactcggcc
ttcaggggtttctgatcgcttctctggctccaagtctggcaacacggcctccctgaccatctctgggctgcaggctgaggacgaggctgattatta
ttgcagctcatattcaagcggctccacacctgctctgtttggggggggcacccagctgaccgtcctc (SEQ ID NO:39);

Light Chain: gatatcgtgctgactcagtcggcctccgtgtctgggtctcctggacagtcgatcaccatctcctgcactggaacca
gcagtgacgttggtggatataactatgtctcctggtaccaacaacacccaggcaaagcccccaaactcatgatttatgatgtcactactcggcctt
caggggtttctgatcgcttctctggctccaagtctggcaacacggcctccctgaccatctctgggctgcaggctgaggacgaggctgattattatt
gcagctcatattcaagcggctccacacctgctctgtttggggggggcacccagctgaccgtcctc (SEQ ID NO:40).

The encoding nucleotide sequences for the variable regions of TRL1230 are:

Heavy Chain: caggtgcagctggtgcagtctgggggaggcctggtcaagcctggggggtccctgagactctcctgtggagcc
tctggatttaacctcagtagttatagcatgaactgggtccgccaggctccagggaaggggctggagtgggtctcatccattagtagtagaagta
gttacatatactatgcagactcagtgcagggccgattcaccatctccagagacaacgccaagaactcactgtatctgcaaatgaacagcctgag
agccgaggacacggctatatattactgtgcgagagtatctccgtccacctattattattatggtatggacgtctggggccaagggaccacggtca
ccgtctcctca (SEQ ID NO:41);

Light Chain: gatatcgtactcactcagccgtcctcggtgtcagtgtccccaggacagacggccaggatcacctgctctggagat
gaattgccaaagcaatatgcttattggtaccagcagaagccaggccaggcccctgtgttggtaatatataaagacaatgagaggccctcaggg
atccctgagcgattctctggctccagctcagggacaacagtcacgttgaccatcagtggagtccaggcagaagacgaggctgactattactgtc
aatcagcagacagtagtggtacttatgtggtgttcggcggagggaccaagctgaccgtccta (SEQ ID NO:42).

The encoding nucleotide sequences for the variable regions of TRL1232 are:

Heavy Chain: caggtgcagctggtggagtctggggctgaggtgaagaagcctggggccttagtgaaggtctcctgcaaggct
tctggatacaccttcagcggctactatatgcactgggtgcgacaggcccctggacaagggcttgagtggatgggatggatcaaccctaagagt
ggtggcacaaagtatgcacagaagtttcagggccgggtcaccatgaccagggacacgtccatcagcacagcctacatggagttgagcaggc
taagatctgacgacacggccgtgtatttctgtgcgagaggcggaccttcaaatttggaacgatttttggagaggttacaaccccgctacagttac
gacgacaagtatgctatggacgtctggggccaagggaccacggtcaccgtctcctca (SEQ ID NO:43);

Light Chain: gatatcgtgatgacccagtctccaggcaccctgtctttgtctccaggggcaagagccaccctctcctgcagggcc
agtcagagtgttagcagcatctatttagcctggtaccagcagaaacctggccaggctcccaggctcctcatctttggtgcatccagcagggcca
ctggcatcccagacaggttcagtggcagtgggtctgggacagacttcactctcaccatcagcagactggagcctgaagattttgcagtgtattac
tgtcagcagtatggtagctcaccgtacacttttggccaggggaccaagctggagatcaaacgaa (SEQ ID NO:44).

The encoding nucleotide sequences for the variable regions of TRL1242 are:

Heavy Chain: caggtgcagctggtgcagtctggaacagaagtgaaaaagcccggggagtctctgaagatctcctgtgagggtt
ctcgatacaactttgccaggtactggatcggctgggtgcgccagatgcccggaaaaggcctggactggatggggatcatctatcctggtgact
ccgataccagatacagcccgtccttccaaggccaggtcagcatctcagccgacaagtccatcagtaccgcctacctgcagtggaacagcctg
aaggcctcggacaccgccatgtattattgtgcgagacttgggagcgagcttggagtggtctctgattattactttgactcctggggccagggaac
cctggtcaccgtctcctca (SEQ ID NO:45);

Light Chain: gatatcgtgttgactcagtctccagactccctggctgtgtctctgggcgagagggccaccatcaactgcaagtcc
agccagagtgttttagacaggtccaacaataagaactgtgtagcttggtaccagcagaaaccgggacagcctcctaaactgctcatttaccggg
ctgctacccgggaatccggggtccctgatcgattcagtggcagcgggtctgggacagacttcagtctcaccatcagcagcctgcaggctgaa
gatgtggcagtttatttctgtcagcaatattatagtattccgaacacttttggccaggggaccaagctggagatcaaacga
(SEQ ID NO:46).

The encoding nucleotide sequences for the variable regions of TRL1245 are:

Heavy Chain: caggtgcagctggtggagtctgggggaggcttggtcaaggctggagggtccctgagactctcctgtgtagcct
ctggattcaccttcagcgactactacatgtcctggattcgccaggctccagggaaggggctggagtggatttcatttattagtagtagtggtgata
ccatattttacgcagactctgtgaagggccgattcaccgtctccagggacagcgccaagaactcactgtatcttcaaatgaacagcctgaaagtc
gaggacacggccgtgtattactgtgcgaggaagggggtgtccgacgaggaactactgcgcttctggggccagggaaccctggtcaccgtct
cctca (SEQ ID NO:47);

Light Chain: gatatcgtgctgactcaggacccctcggtgtcagtgtccccaggacaaacggccaggatcacctgctctggaga
tgcattgccaaaaaaatatgcttattggtaccagcagaagtcaggccaggcccctgtgctggtcatctatgaggacaccaaacgaccctccgg
gatccctgagagattctctggctccagctcagggacagtggccaccttgactatcagtggggcccaggtggaggatgaagctgactactattgt
tactcaacagacagcagcggtaatcagagggtattcggcggagggaccaagctgaccgtccta (SEQ ID NO:48).

### Example 2

### Determination of Affinity

For practice of the assay method, ~1 mg of IHF was required. IHF is difficult to express in bacteria (since it has a dual function involving gene regulation, leading to toxicity to bacteria expressing high levels). Obtaining sufficient material for mAb discovery from bacterial sources is thus difficult (and expensive). The protein was therefore expressed in HEK293 (mammalian) cells, with a poly-histidine tag to enable easy purification. The homologs from *Staphylococcus aureus* (Sa), *Pseudomonas aeruginosa* (Pa), *Klebsiella pneumoniae* (KP) and *Haemophilus influenzae* (Hi) were all prepared in this manner. These four are of particular utility since they span a substantial portion of the diversity in sequences of the DNABII family.

TRL295 was shown to bind with high affinity to the IHF peptide of *H. influenzae* and moreover to bind to IHF from additional bacterial species.

The chart below shows the degree of identity to *Haemophilus* of various IHF and HU proteins from a variety of bacterial species.

| Species | Protein | Sequence Identity to *Haemophilus* |
|---|---|---|
| *Haemophilus influenzae* | IHF alpha | 100 |
| *Escherichia coli* | IHF alpha | 67 |
| *Enterobacter cloacae* | IHF alpha | 66 |
| *Enterobacter aerogenes* | IHF alpha | 66 |
| *Klebsiella oxytoca* | IHF alpha | 65 |
| *Pseudomonas aeruginosa* | IHF alpha | 61 |
| *Acinetobacter baumannii* | IHF alpha | 58 |
| *Streptococcus pneumoniae* | HU | 38 |
| *Staphylococcus aureus* | HU | 38 |

Further, the high affinity binding of TRL295 was shown to be retained even as the pH was decreased from physiological (pH 7.5) to pH 4.5, as shown below.

| pH | Kd (nM) |
|---|---|
| 7.5 | 4.2 |
| 6.5 | 2.8 |
| 5.5 | 2.8 |
| 4.5 | 3.7 |
| 3.5 | no binding |
| 2.5 | no binding |

This is important since bacteria often secrete lactic acid which reduces the local microenvironment pH as a way of inhibiting immune system attack.

The chart below shows the results of ELISA assays to determine binding of various DNABII proteins. The numbers represent OD values which are useful for comparison to TRL1068 - higher values represent higher binding affinity. TRL1068 shows similar binding to all four homologs, but low binding to BSA, as does TRL1215. The abbreviations are
Hi = *Haemophilus influenzae*; Kp = *Klebsiella pneumoniae*;
Pa = *Pseudomonas aeruginosa*; Sa = *Staphylococcus aureus*

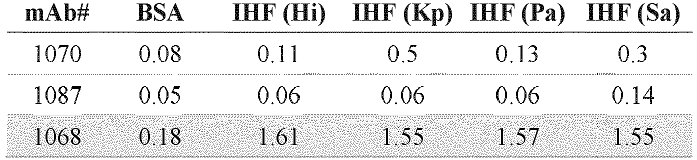

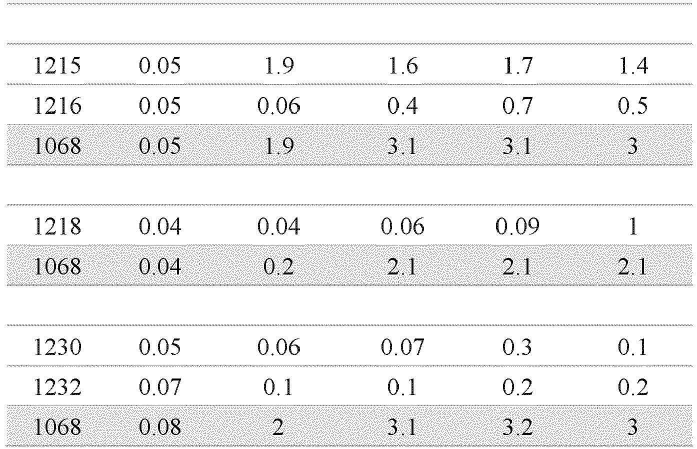

The affinity of TRL1068 for the target protein was directly determined using a ForteBio Octet^{™} biosensor model QK (Pall Corporation; Menlo Park, CA) with Kd determined by standard methods for measuring ratio of on and off rates (Ho D, et al., BioPharm International (2013) 48-51). The values were: 1 nM for *Staphylococcus aureus* (Sa), 1 nM for *Pseudomonas aeruginosa* (Pa), 7 nM for *Klebsiella pneumoniae* (Kp) and 350 nM for *Haemophilus influenzae* (Hi).

### Example 3

### Epitope Selection for Focused mAb Discovery

Computational methods for analyzing the likelihood of antigenicity (induction of antibody responses) are known in the art (reviewed by J. Ponomarenko, et al., in BMC Bioinformatics (2008) 9:514). Using an improved variation of these published methods, a map of the likely epitopes was generated for the IHF from *Haemophilus influenzae* from a model of the structure based on the published structure found in the Protein Data Bank (pdb 1OWF) (Figure 1B). For the display in Figure 1A, a value was assigned to the residue at the midpoint of each 11-amino acid segment. A value above 0.9 denotes a region with high likelihood of being susceptible to antibody binding.

Three regions were identified as having high likelihood of being recognized by antibodies: residues 10-25, 56-78, and 86-96.

As illustrated in Figure 2, the region from residues 56-78 of the IHF protein is substantially conserved across multiple clinically important bacterial species. Structural modeling of IHF from multiple species has confirmed that the homology is high, particularly in the DNA binding region (Swinger, K. K., et al., Current Opinion in Structural Biology (2004) 14:28-35). Peptides that only partially overlap with this optimal region are less likely to fold spontaneously into the relevant three dimensional conformation and will be more difficult to chemically crosslink in order to lock in that conformation. Optimizing the fidelity to the native protein in this manner is advantageous for both mAb discovery and for use of the peptide as an immunogen.

As noted above, the epitopes thus identified are positions 10-25 of *H. influenzae* IHF: IEYLSDKYHLSKQDTK (SEQ ID NO:49); positions 56-78 of *H. influenzae* IHF: RDKSSRPGRNPKTGDVVAASARR (SEQ ID NO:50); and positions 86-96 of *H. influenzae* IHF: QKLRARVEKTK (SEQ ID NO:51).

Figure 3A shows a computational construction of the IHF dimer complexed with DNA. The B cell epitopes of the invention are shown in Figure 3B. Figure 3A shows that the epitopes are partially masked by DNA when bound. However, if exposed, these portions of the proteins may generate antibodies of high affinity capable of binding them and thus preventing the formation of biofilm or causing an established biofilm to lose structural integrity as the DNABII protein is sequestered by the antibody. Other sites on the DNABII protein may also suffice to achieve extraction of the protein out of the biofilm based on higher affinity binding by the mAb as compared to the protein's affinity for components of the biofilm.

### Example 4

### In Vitro Bioactivity Assessment

TRL1068 was tested for bioactivity using a commercial assay from Innovotech (Edmonton, Alberta; Canada). Biofilms were formed in multiple replicates on pins in a 96-well microplate format exposed to media including *Pseudomonas aeruginosa* (ATCC 27853) or *Staphylococcus aureus* (ATCC 29213). Following biofilm formation, the pins were treated in different wells with a non-immune isotype control mAb or with TRL1068 at 1.2 µg/mL (~10 nM) for 12 hours. As evident in the scanning electron micrographs of the treated surfaces in Figure 4, TRL1068 was highly effective at dissolving the biofilm. These results establish that the mAb can degrade the biofilm, thereby removing the attached bacteria.

### Example 5

### In Vivo Bioactivity Assessments

Several animal models exist for evaluation of activity. For example, at University Hospital Basel (Switzerland), a model for biofilm on implanted prostheses involves implanting Teflon^{®} tissue cages (Angst+Pfister; Zurich, Switzerland) subcutaneously in BALB/c mice, which are then allowed to heal for 2 weeks. After confirming sterility of the cage by extracting fluid from it, the site is infected with 4 × 10³ CFU (colony-forming units) of *S. aureus* (ATCC 35556), an inoculum mimicking a perioperative infection. After 24 hours, the site is injected with drug. After 72 hours, the mice are sacrificed and the tissue cage recovered. Viable bacteria are counted by plating on blood agar (Nowakowska J., et al., Antimicrob Agents Chemother (2013) 57:333).

A second example is a model that involves inducing biofilm on heart valves, mimicking native valve endocarditis (Tattevin P., et al., Antimicrob Agents Chemother (2013) 57:1157). New Zealand white rabbits are anesthetized. The right carotid artery is cut and a polyethylene catheter is positioned across the aortic valve and secured in place. Twenty four hours later, 1 mL of saline plus 8 × 10⁷ CFU of *S. aureus* is injected through the catheter, which induces a biofilm infection in 95% of the animals. Drugs (anti-biofilm and antibiotic) are administered i.v. and efficacy is evaluated after 4 days by tissue pathology and blood bacterial levels.

## Claims

1. A monoclonal antibody (mAb) which mAb has affinity for at least one DNABII protein that exceeds the affinity of said protein for components of a biofilm that includes said DNABII protein, which mAb includes an antigen binding fragment, an Fv antibody a bispecific antibody, and a complete antibody;
wherein the variable region of said mAb comprises
the variable region of the heavy chain of TRL1068 of SEQ ID NO:5 and further comprises the variable region of the light chain of TRL1068 of SEQ ID NO:6.

2. The mAb of claim 1 wherein the DNABII protein is IHF.

3. The mAb of claim 1 which is an mAb which is a human mAb.

4. An *in vitro* method to prevent formation of or to dissolve a biofilm associated with an industrial process which method comprises treating a surface susceptible to or containing a biofilm with the mAb of any of claims 1-3.

5. Recombinant host cells that have been modified to contain a recombinant expression system for producing the mAb of any of claims 1-3 wherein the binding moiety is a mAb wherein said expression system comprises a nucleotide sequence encoding said mAb operably linked to control sequences for expression.

6. A method to prepare an mAb that binds a DNABII protein which method comprises culturing the cells of claim 5 and recovering said mAb.

7. The mAb of any of claims 1-3 for use in a method to treat a condition in a subject **characterized by** the formation of a biofilm in said subject.

8. The mAb for use of claim 7 wherein said condition is heart valve endocarditis, chronic non-healing wounds, including venous ulcers and diabetic foot ulcers, ear infections, sinus infections, urinary tract infections, pulmonary infections, cystic fibrosis, chronic obstructive pulmonary disease, catheter-associated infections, infections associated with implanted prostheses, and periodontal disease.

## Patentansprüche

1. Monoklonaler Antikörper (mAb), wobei der mAb eine Affinität für mindestens ein DNABII-Protein hat, welche die Affinität des Proteins für Komponenten eines Biofilms, der das DNABII-Protein einschließt, übersteigt, wobei der mAb ein Antigen-bindendes Fragment, einen Fly-Antikörper, einen bispezifischen Antikörper, und einen vollständigen Antikörper einschließt;
wobei die variable Region des mAb die variable Region der schweren Kette von TRL1068 von SEQ ID NO:5 umfasst und ferner die variable Region der leichten Kette von TRL1068 von SEQ ID NO:6 umfasst.

2. mAb nach Anspruch 1, wobei das DNABII-Protein IHF ist.

3. mAb nach Anspruch 1, der ein mAb ist, der ein menschlicher mAb ist.

4. *In-vitro* Verfahren, um die Bildung eines Biofilms zu verhindern oder einen Biofilm aufzulösen, der mit einem industriellen Prozess assoziiert ist, wobei das Verfahren Behandeln einer Oberfläche, die für einen Biofilm anfällig ist oder einen Biofilm enthält, mit dem mAb nach einem der Ansprüche 1-3 umfasst.

5. Rekombinante Wirtszellen, die modifiziert worden sind, um ein rekombinantes Expressionssystem zum Herstellen des mAb nach einem der Ansprüche 1 - 3 zu enthalten, wobei die Bindungseinheit ein mAb ist, wobei das Expressionssystem eine Nukleotidsequenz umfasst, kodierend den mAb, funktionell verbunden mit Kontrollsequenzen für Expression.

6. Verfahren, um einen mAb herzustellen, der ein DNABII-Protein bindet, wobei das Verfahren Kultivieren der Zellen nach Anspruch 5 und Gewinnen des mAb umfasst.

7. mAb nach einem der Ansprüche 1 - 3 zur Verwendung in einem Verfahren um einen Zustand in einem Subjekt, charakterisiert durch die Bildung eines Biofilms in dem Subjekt, zu behandeln.

8. mAb zur Verwendung nach Anspruch 7, wobei der Zustand Herzklappenendokarditis, chronische nicht-heilende Wunden, einschließlich venöser Geschwüre und diabetischer Fußgeschwüre, Ohrinfektionen, Sinusinfektionen, Harnwegsinfektionen, Lungeninfektionen, zystische Fibrose, chronisch obstruktive Lungenerkrankung, Katheter-assoziierte Infektionen, Infektionen, assoziiert mit implantierten Prothesen, und Parodontalerkrankungen ist.

## Revendications

1. Anticorps monoclonal (mAb), lequel mAb a une affinité pour au moins une protéine DNABII qui dépasse l'affinité de ladite protéine pour des composants d'un biofilm qui contient ladite protéine DNABII, lequel mAb contient un fragment de liaison à un antigène, un anticorps Fv et un anticorps bispécifique, et un anticorps complet ;
dans lequel la région variable dudit mAb comprend la région variable de la chaîne lourde de TRL1068 ayant la SEQ ID NO : 5 et comprend en outre la région variable de la chaîne légère de TRL1068 ayant la SEQ ID NO : 6.

2. mAb selon la revendication 1, dans lequel la protéine DNABII est IFH.

3. mAb selon la revendication 1, qui est un mAb humain.

4. Méthode *in vitro* pour dissoudre ou empêcher la formation d'un biofilm associé à un procédé industriel, laquelle méthode comprend le traitement d'une surface susceptible de contenir ou contenant un biofilm avec le mAb de l'une quelconque des revendications 1 à 3.

5. Cellules hôtes recombinées qui ont été modifiées de manière à contenir un système d'expression recombiné pour produire le mAb de l'une quelconque des revendications 1 à 3, dans lesquelles le fragment de liaison est un mAb, dans lesquelles ledit système d'expression comprend une séquence de nucléotides codant ledit mAb liée de manière fonctionnelle à des séquences de contrôle pour l'expression.

6. Méthode pour préparer un mAb qui se lie à une protéine DNABII, laquelle méthode comprend la culture des cellules de la revendication 5 et la récupération dudit mAb.

7. mAb selon l'une quelconque des revendications 1 à 3, pour une utilisation dans une méthode pour traiter un état chez un sujet, **caractérisé par** la formation d'un biofilm dans ledit sujet.

8. mAb pour une utilisation selon la revendication 7, dans lequel ledit état est une endocardite valvulaire, une plaie chronique ne cicatrisant pas, y compris des ulcères veineux et des ulcères aux pieds diabétiques, des infections auriculaires, des infections des sinus, des infections des voies urinaires, des infections pulmonaires, une mucoviscidose, une broncho-pneumopathie chronique obstructive, des infections associées à un cathéter, des infections associées à des prothèses implantées, et une maladie parodontale.
